# EUROPEAN PATENT APPLICATION

(11) **EP 4 841 260 A1**
(43) Date of publication of application: **18.11.2026**
(21) Application number: 25176575.6
(22) Date of filing: 15.05.2025
(51) Int. Cl.: G01N 33/543, B01L 3/00, G01N 1/38

(54) **CARTRIDGE AND METHOD FOR DILUTION OF TARGET ANALYTE**

(71) Applicant: Siemens Healthineers Nederland B.V., 2595 BN Den Haag (NL)
(72) Inventor: De Theije, Femke Karina, 5351 LB Berghem (NL); Dekkers, Dave, 3755SL Eemnes (NL); Peeters, Emiel, 5655JV Eindhoven (NL)
(74) Representative: Siemens Healthineers Patent Attorneys

(57) **Abstract**

The present invention relates to a cartridge for the uptake and analysis of a fluid sample with respect to the presence and/or quantity of a target, comprising one or more reaction zones and one or more transport zones, which zones are designed for contacting of reagents with a sample, wherein said one or more zones comprise as reagent one or more free first interactor(s) of said target. Further envisaged is a system comprising the cartridge and a handheld device. Moreover, the present invention is directed to a method of reducing the active concentration of a target in a fluid sample as well as a use of the cartridge or system for immunological assays.

## Description

### TECHNICAL FIELD

The present invention relates to a cartridge for the uptake and analysis of a fluid sample with respect to the presence and/or quantity of a target, comprising one or more reaction zones and one or more transport zones, which zones are designed for contacting reagents with a sample, wherein said one or more zones comprise as reagent at least one free first interactor of said target. Further envisaged is a system comprising the cartridge and a handheld device. Moreover, the present invention is directed to a method of reducing the active concentration of a target in a fluid sample as well as a use of the cartridge or system for immunological assays.

### BACKGROUND

Point Of Care Testing (POCT) can provide results to a physician more rapidly than routine central lab testing. Thus, substituting central lab testing with that performed at the Point of Care (POC) is considered to increase speed and efficiency of diagnostic approaches. In particular, for patient safety, proper analytical validation and establishing high-sensitivity performance is critical. Handheld devices which integrate several functions, such as sample preparation, analyte detection and signal processing, are hence of paramount importance for medical practitioners, in particular in remote locations without central lab environments or in acute care settings where there is an immediate need for test results to aid in clinical decision making. The POCT systems typically need to be easy to use and should require only simple sample handling steps since users may not be highly trained or skilled. This means that no pretreatment steps can be applied to the sample before application into a cartridge.

One of the very common pretreatment steps in lab diagnostics is dilution of the sample. If the sample concentration is too high, it can lead to signal saturation, making it difficult to quantify the amount accurately. Dilution prevents signal saturation and ensures that the target molecule concentration falls within the assay's dynamic range, where the signal is reliably proportional to concentration. Dilution also prevents a high-dose hook (HDH) effect (or "prozone effect") which can cause falsely low readings in immunoassays when the target analyte is present at very high concentrations. In immunoassay testing this occurs because an excess of the target molecules can saturate both the capture and detection antibodies, preventing proper formation of the antibody-analyte complex needed for signal generation. By dilution it can be made sure that the measurement falls within the assay's dynamic range. However, since pretreatment steps cannot be performed in POCT approaches, there is a need for an alternative for dilution activities.

### SUMMARY

The present invention addresses this need and provides a cartridge for the uptake and analysis of a fluid sample with respect to the presence and/or quantity of a target, the cartridge comprising one or more reaction zones and one or more transport zones, which zones are designed for contacting reagents with a sample, wherein said zones comprise as reagent at least one free first interactor of said target. The described cartridge advantageously allows said first interactor(s) to catch and inactivate a fraction of the target molecules in the reaction and transport zones and thereby "dilute" the target molecule concentration. Thereby the measuring range of the test can be significantly extended. Further, the HDH effect can advantageously be delayed. A further advantage is that the dynamic range of the assay can be extended.

In a preferred embodiment of the present invention, the free first interactor of said target is a molecule specifically binding to said target.

In a further preferred embodiment, the cartridge further comprises as reagent a second and, optionally, a further interactor of said target which second and further interactors are designed for detecting and/or capturing the target and wherein the free first interactor (i) binds in the same binding region of the target as the second or the further interactor; or (ii) binds at any region of the target and thereby prevents the binding of said second and/or further interactors.

In a particularly preferred embodiment, the specifically binding molecule is an antibody, an antibody substructure, or a modified antibody, or a fragment thereof; or a non-immunoglobulin protein selected from the group of: designed ankyrin-repeat protein (DARPin), affimer, aptamer, affibody molecule, adnectin, anticalin, affilin, avimer, knottin, fynomer, phylomer, artificial antibody mimic and kunitz domain peptide.

In a further preferred embodiment, the free first interactor is provided as dried reagent in the zones of the cartridge as mentioned above. It is particularly preferred that the free first interactor is not immobilized on a bead or a surface of the cartridge.

In yet another preferred embodiment, the free first interactor is provided as liquid reagent in the zones of the cartridge as mentioned above.

In yet another embodiment, the free first interactor is provided at the place of first contact of the fluid sample with reagents in the cartridge. It is particularly preferred that the free first interactor is provided in a mixture with a buffer or buffer system.

In a further aspect the present invention relates to a system comprising a handheld device capable of operating a cartridge according to the invention and a cartridge according to the invention as defined herein.

In a further aspect, the present invention relates to a method of reducing the active concentration of a target in a fluid sample, the method comprising the step of contacting at least one free first interactor of the target with the fluid sample to allow the binding of said at least one free first interactor to the target, thereby reducing the active concentration of the target in the sample.

In a further aspect, the present invention relates to a method of analyzing a fluid sample with respect to the presence and/or quantity of a target, the method comprising a step of reducing the active concentration of a target in a fluid sample according to the method as defined above and wherein the method comprises the additional step of analyzing said fluid sample with respect to the presence and/or quantity of the target, preferably by using a second and, optionally, a further interactor which second and further interactors are designed for detecting and/or capturing the target.

In particularly preferred embodiments of the methods, the free first interactor, the second and/or further interactor of the target is a molecule specifically binding to said target, preferably an antibody, an antibody substructure, or a modified antibody, or a fragment thereof; or a non-immunoglobulin protein selected from the group of: designed ankyrin-repeat protein (DARPin), affimer, aptamer, affibody molecule, adnectin, anticalin, affilin, avimer, knottin, fynomer, phylomer, artificial antibody mimic and kunitz domain peptide.

In further preferred embodiments, the methods as described above additionally comprise a step of calibrating one or more reagents, preferably in a cartridge according to the invention as described herein.

In further preferred embodiments of the methods as described above, the binding of said at least one free first interactor to said target is performed for about 1 to 15 minutes as of the first contact of the fluid sample with the at least one free first interactor.

In a further aspect, the present invention relates to the use of the cartridge or the system according to the present invention as defined herein for molecular diagnosis, immunological assays, biological or chemical sample analysis, body fluid sample analysis, preferably blood sample analysis, saliva sample analysis, intestinal fluid sample analysis, eye fluid sample analysis, urine sample analysis, pharmacology, food analysis, environmental, veterinary or forensic analysis.

In a particularly preferred embodiment, the fluid sample is a blood or blood plasma sample, a saliva sample, an intestinal fluid sample, a urine sample or an eye fluid sample.

### BRIEF DESCRIPTION OF THE DRAWINGS

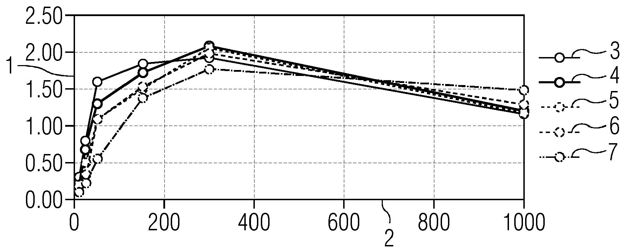

FIG. 1 shows results of an experiment evaluating the effect of addition of free first interactor (C6cc antibody) at different concentrations on the dose response curve of a CRP assay according to an embodiment of the present invention. The figure shows the recorded output signal of the assay (signal ratio (1) ) as function of the CRP target concentration in mg/mL (2). Each curve relates to a different concentration of free C6cc antibody. At low concentrations of C6cc antibody, the signal is already saturated at very low CRP concentrations and a decline of output signal is observed with increasing CRP concentrations (high-dose Hook effect). At higher C6cc antibody concentrations the signal saturates at a higher CRP concentration, thereby extending the dynamic range of the assay, and the signal does not show the decline of signal at higher CRP dose. Depicted are C6cc antibody concentrations of 0.25 (3), 0.30 (4), 0.35 (5), 0.40 (6) and 0.50 (7) mg/mL.

### DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "(i)", "(ii)", "(iii)" or "(a)", "(b)", "(c) ", "(d) ", or "first", "second", "third" etc. and the like in the description or in the claims, are used for distinguishing between similar elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e. the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

Independent of the grammatical term usage, individuals with male, female or other gender identities are included within the term.

As has been set out above, the present invention concerns in one aspect a cartridge for the uptake and analysis of a fluid sample with respect to the presence and/or quantity of a target, the cartridge comprising one or more reaction zones and one or more transport zones, which zones are designed for contacting reagents with a sample, wherein said zones comprise as reagent at least one free first interactor of said target.

The term "cartridge for the uptake and analysis of a fluid sample" as used herein refers to a structure or an instrument, or part of an instrument, or a part of a system, which allows or is suitable for the performance of reactions, in particular molecular reactions involving chemical and/or biological entities and/or particle counting activities and/or the measurement of physical and/or chemical parameters. It is preferred that the reaction includes a coordination between an interactor such as an antibody and its binding partner such as antigen on a target, e.g. in the formation of a sandwich assay. The cartridge may correspondingly be equipped, for example, with a suitable inlet element, it may comprise one or more surfaces, e.g. reactive surfaces or surfaces with specific functionality, it may comprise, for example, a washing zone, a mixing zone, a waiting zone, a measurement zone, a waste zone, a reservoir zone, a recollection or a regeneration zone etc. or any sub-portion or combination thereof. In specific embodiments, the cartridge may comprise a filter zone comprising a filter membrane which allows for separating different components within the sample and for adding reagents to the sample; a landing zone which allows for delaying the movement of the sample and for blending the sample; one or more transport zones which allow for transporting the sample from, e.g. the landing zone to a reaction zone, comprising one or more fluidic channels; and one or more reaction zones.

The cartridge comprises in particular one or more reaction zones and one or more transport zones. These zones are designed for the contacting or mixing of reagents with a sample. The zones may further have additional functions. A reaction zone may, for example, be designed to capture analytes or targets from the sample and other reactants. A reaction zone may alternatively be designed for washing, examination and verification of assay reactions. The reaction zones may, in further embodiments, also comprise or be connected to one or more stop zones where fluidic flow within the cartridge is stopped.

The cartridge may, in additional embodiments, comprise connections between the mentioned elements, e.g. tubes or joints; and/or it may comprise reservoirs and repositories for liquids, fluids, reactants, chemicals, ingredients, samples or any other entity to be used within the cartridge. The zones may further also be equipped with suitable sensor elements allowing the measurement of e.g. light emission, light reflection, light scattering, light interference, temperature changes or temperature gradients.

The cartridge is designed, inter alia, for the analysis of the presence and/or quantity of a target. The term "target" as used herein relates to any type of chemical or biochemical entity of interest, which is amenable to molecular analysis, e.g. immunological analysis. The term refers, inter alia, to small organic molecules, to nucleic acids, e.g., RNA or DNA molecules, or peptides or polypeptides (proteins). Preferably, the target is a peptide or polypeptide (protein) molecule. The target may, for example, be a polypeptide (protein) which is present, under certain conditions, in a fluid sample. The presence and/or quantity of the target may allow for diagnostic conclusions, e.g. with respect to the presence of an illness and/or the stage or severity of the illness etc. In a specific embodiment, the target may be any polypeptide (protein) or peptide which is present in high concentration in blood. In further specific embodiments the target may be C-reactive protein (CRP) , human serum albumin (HAS) or the fibrin degradation product D-Dimer.

The term "presence and/or quantity of a target" as used herein means that the analysis is a qualitative detection, a semi-quantitative detection or a quantitative detection.

The analysis may hence include qualitative, semi-quantitative or quantitative detection. The analysis is typically based on a biological or biochemical assay format as known to the skilled person. Such assays typically comprise the following general steps: sample processing and manipulation, target specific discrimination or identification, signal or target amplification, signal detection and/or interpretation, and signal enhancement and noise filtering. The assays may include end point assays, e.g. assays in which a single measurement is performed after a fixed incubation period, or kinetic assays, i.e. assays in which measurements are performed multiple times over a fixed time interval. The assays may, for example, include assays of the biological activity of a sample, ligand binding assays, where a ligand binds a receptor, or immunoassays, where the response is an antigen-antibody binding type reaction. Particularly preferred are immunologic assays in which a target is detected with a suitable binding molecule. Further envisaged are enzyme assays, where, for example, enzymes may be tested via their highly repeating activity on a large number of substrates when loss of a substrate or the making of a product may have a measurable attribute like color or absorbance at a particular wavelength or light or electrochemiluminescence or electrical/redox activity. Also possible are combinations of any of the above-mentioned assays.

The term "qualitative detection" as used herein means that the analysis aims at indicating whether a specific target is present or not. By detecting, for instance, a signal via a bound antibody, it can be deduced that the target is present in a sample. For example, in an analysis based on an ELISA test a qualitative assay means that the assay can be used to achieve a yes or no answer indicating whether a target (e.g. via specific binding of a specifically binding secondary interactor) is present in a sample. This may include the performance of control or calibration tests.

The term "semi-quantitative detection" as used herein means that the analysis aims at indicating whether a specific target is present above a certain threshold with respect to its numbers or amount or concentration in a fluid sample. The threshold may be suitably defined as would be known to the skilled person. For example, by detecting a signal in an assay on the basis of a second or further interactor as described herein, it can be deduced that the target which is present has surpassed a certain predefined limit with respect to its numbers, amount or concentration. The format also allows to compare relative levels of the target between samples, in particular between samples used within the same assay format.

The term "quantitative detection" as used herein means that the analysis aims at indicating the approximate or exact numbers, amount or concentration of a specific target. For the quantitative detection, suitable control and/or calibration steps are required. Typically, the quantitative detection involves the interpretation of signals in comparison to a standard curve (e.g. a serial dilution of a known, purified target) in order to precisely calculate the concentrations of target in various samples. For example, in an ELISA format, the signal data obtained may be graphed with optical density vs. log concentration to produce a sigmoidal curve. Known concentrations of target may be used to produce a standard curve. This data may subsequently be used to measure the concentration of unknown samples by comparison to the linear portion of the standard curve. This can advantageously be done directly on the graph or with suitable curve fitting software known to the skilled person.

The term "fluid sample" as used herein relates to any inorganic or organic liquid sample. Preferably, the sample is a biological sample. The term "biological sample" as used herein refers to any specimen obtained from a biological organism, preferably a living organism. The term relates also to specimen obtained from non-living, i.e. dead biological organisms, in particular recently deceased organisms. The term "biological organism" includes in general eukaryotic systems and may also comprise sub-portions of eukaryotic systems. In particular, such organisms include higher eukaryotes. In preferred embodiments of the present invention, a biological sample may be derived from an animal, preferably from a mammal, e.g. from a cat, a dog, a swine, a horse, a cattle, a sheep, a goat, a rabbit, a rat, a mouse, or a monkey. Particularly preferred is a sample obtained from a human being. The sample may be obtained via suitable methods known to the person skilled in the art. The sample used in the context of the present invention should preferably be collected in a clinically acceptable manner, more preferably in a way that proteins are preserved.

The sample may include body fluids, such as blood, sweat, sputum or saliva, semen and urine. Particularly preferred are body fluid samples such as whole blood, blood plasma, saliva, intestinal fluid, urine, or eye fluid.

In certain embodiments the sample may be a quality control sample or a calibration sample. These samples may comprise a predefined or known content, e.g. a predefined or known concentration of the target.

In specific embodiments, a cartridge according to the present invention may be calibrated by measuring samples with known concentration and recording the output signal for each sample. Thereby a calibration curve can be obtained which can be linked electronically to each cartridge. This curve may subsequently be used to link the measurement result of an unknown sample to a dose or specific value on this curve. The calibration may, for example, be performed for larger groups of cartridges, e.g. a cartridge lot. The link to each cartridge may, in specific embodiments, be provided by programming the obtained curve onto an RFID chip which may be included in the cartridge.

The term "reagent" as used herein relates to reactants or ingredients which are involved in the performance of a molecular analysis, preferably of a diagnostic analysis. Such elements may comprise, for example, a buffer, a peptide, a polypeptide (protein), an enzyme, a nucleotide, an antibody, or a dye or any other ingredient necessary for the analysis. The reagent may be provided in any suitable form, e.g. as liquid or in a dried or lyophilized form. In specific embodiments, during manufacture of a cartridge, all reagents used in the context of the present invention are provided by dosing of a liquid containing the reagents. Subsequently, the liquid is dried in the cartridge and thereby the reagents are left on the surface of the cartridge in dried form, e.g. in one or more zones of the cartridge as defined herein.

According to the invention, the one or more reaction zones and one or more transport zones comprise as reagent a free first interactor. The term "free first interactor" as used herein refers to any chemical or biochemical entity which is capable of interacting with the target, wherein said interactor is not tethered or immobilized or bound at a specific location in the mentioned zones, but non-immobilized and unbound, e.g. free floating in a liquid or dissolvable into a non-immobilized interactor, e.g. after initially dried reagents are dissolved, e.g. by the sample. In certain embodiments, the free first interactor may be a group or mixture of different free interactors which are capable of binding to the target. The free first interactor may thereby be distinguished from a "second interactor" or a "further interactor", e.g. a third interactor etc., which may be capable of binding the same target, but are designed for detecting and/or capturing a target, being typically immobilized at one or more sites of the zones as mentioned above, preferably at one or more reaction zones or being labeled with a dye. In specific embodiments, the second interactor or further interactor, e.g. third interactor, may be labeled, e.g. with a suitable dye, preferably with a chemiluminescent dye. In preferred embodiments, a sandwich assay may be performed in which a second interactor is immobilized and is not labeled and a further, e.g. third interactor, may not be immobilized, but be labeled, e.g. with a chemiluminescent dye, thus allowing for detection of the target.

It is particularly preferred that the first and second or further interactors are in competition with each other. The competition may be implemented by providing the first and the second, and, optionally, any further interactor at the same time. Alternatively or in addition, the competition may be implemented by providing the first and the second, and, optionally, any further interactor, at the same location and the same time.

The term "chemiluminescent dye" as used herein refers to an agent which, upon a chemical reaction, emits electromagnetic radiation in the ultraviolet or visible light range. Examples of chemiluminescent dyes include luminol, imidazole, or acridinium esters. Particularly preferred is the use of acridinium esters (AE). Acridinium esters are currently the most commonly used direct chemiluminescent dyes since they advantageously show high stability, low background, high signal-noise ratio, quick light-emitting and a high luminous efficiency. Typically, AE independently emit light in the presence of H₂O₂ and NaOH without the participation of any enzyme and reach their maximum chemiluminescence in about 0.4 seconds. Thus, exposing the AE to an alkaline H₂O₂ solution typically triggers a flash. Before the flash occurs, the acridone, which is the light emitting species, separates from the remainder of the conjugate, so the subsequent light emission is not influenced by further components. It is preferred that the trigger solution comprises a detergent, which improves the reaction speed. Particularly preferred is the detergent ARQUAD 16-50.

Preferred is the use of chemiluminescent dyes which emit light in different wavelengths, preferably in the range of 400 to 430 nm, e.g., at 425 nm; 450 to 500 nm, e.g., 475 nm, 500 to 550 nm, e.g., 525 nm; 600 to 650 nm, e.g., 625 nm or 675 to 725 nm, e.g., 700 nm. Preferred is the employment of acridinium ester chemiluminescent dyes such as DMAE, NSP-DMAE, HEGAE, HQYAE, ZAE, TSPAE, NSP-LEZAE, Rhodamine-2-AM-DMAE or CNF-2-AM-DMAE, or analogues thereof. Particularly preferred is the use of NSP-DMAE, HQYAE, TSPAE, NSP-LEZAE, Rhodamine-2-AM-DMAE or CNF-2-AM-DMAE. NSP-DMAE is a chemiluminescent dye which emits light with a peak wavelength of 427 nm. TSPAE is a chemiluminescent dye which emits light with a peak wavelength of 480 nm. HQYAE is a chemiluminescent dye which emits light with a peak wavelength of 480 nm. NSP-LEZAE is a chemiluminescent dye which emits light with a peak wavelength of 522 nm. Rhodamine-2-AM-DMAE is a chemiluminescent dye which emits light with a peak wavelength of 620 nm. CNF-2-AM-DMAE is a chemiluminescent dye which emits light with a peak wavelength of 718 nm. Also envisaged are analogues of said AEs or other chemiluminescent dyes, including future developments of these or similar dyes.

It is preferred that the free first interactor of the target is a molecule specifically binding to said target. The free first interactor reduces the number or concentration of available sites or regions for interaction with specific binding molecules on the target. In one embodiment, said free first interactor binds in the same binding region of the target as a second or further interactor as defined herein above, i.e. an interactor designed for detecting and/or capturing the target. In a further embodiment, the free first interactor may bind at any region of the target. The binding may, e.g. by steric hinderance or 3D structure modifications or conformational changes, prevent the binding of said second or further interactor, thereby preventing detecting and/or capturing the target bound by the second or further interactor. Hence, the amount and/or concentration of targets available for interaction, e.g. unhindered binding, with said second or further interactor is reduced, which results in an effect comparable to a dilution of the target.

According to preferred embodiments, said specifically binding molecule, e.g. free first or second or further interactor, is an antibody, an antibody substructure, or a modified antibody, or a fragment thereof; or a non-immunoglobulin protein selected from from the group of: designed ankyrin-repeat protein (DARPin), affimer, aptamer, affibody molecule, adnectin, anticalin, affilin, avimer, knottin, fynomer, phylomer, artificial antibody mimic and kunitz domain peptide.

The term "antibody" as used herein refers to immunoglobulin molecules and immunologically active portions of immunoglobulin molecules, i.e. molecules that contain an antigen binding site that specifically binds an antigen. The immunoglobulin molecules of the invention can be of any type (e.g., IgG, IgE, IgM, IgD, IgA and IgY), class (e. g., IgG1, IgG2, IgG3, lgG4, lgA1 and IgA2) or subclass of immunoglobulin molecules. The antibody may be a polyclonal, monoclonal, multi-specific, human, humanized or chimeric antibody. It is preferred that the antibody is a monoclonal antibody. Also envisaged are antibodies from mouse, rabbit, goat, sheep, bovine or other suitable animals.

The term "antibody substructure" as used herein refers to single chain antibodies, Fab fragments, Fab' fragments, fragments produced by a Fab expression library, F(ab')2, Fv, disulfide linked Fv, minibodies, diabodies, scFv, sc(Fv)2, and epitope-binding fragments of any of the above. Preferred are Fab, Fab' and F (ab')2, Fv, single-chain Fvs (scFv), sc(Fv)2, single-chain antibodies, disulfide-linked Fvs (sdFv) and fragments comprising either a VL or VH domain. The term "Fab fragment" as used herein refers to antibody fragments consisting of the first constant domain of the heavy chain (CH1), the constant domain of the light chain (CL), the variable domain of the heavy chain (VH) and the variable domain of the light chain (VL) of an intact immunoglobulin protein. Typically, Fab fragments are obtained by proteolytic cleavage by papain. The term "F(ab')2" or "F(ab')2 fragment" as used herein refers to antibody fragments consisting of two first constant domains of the heavy chain (CH1), two constant domains of the light chain (CL), two variable domains of the heavy chain (VH) and two variable domains of the light chain (VL) of an intact immunoglobulin protein, i.e. it comprises two Fab fragments. Additionally, F(ab')2 molecules comprise a S-S linkage in the antibody hinge region which combines the Fab fragments. Typically, F(ab') 2 fragments are obtained by proteolytic cleavage by pepsin. The term "Fab' fragment" as used herein refers to fragments derived from "F(ab')2" molecules, preferably fragments comprising the S-S linkage in the antibody hinge region. The term "Fv fragments" as used herein refers to antibody fragments consisting of the two variable antibody domains VH and VL. The term "single chain Fv fragment (scFv)" as used herein relates to antibody fragments consisting of the two VH and VL domains linked together by a flexible peptide linker. The term "diabody" as used herein refers to an antibody variant comprising a separated VH-VL and VL-VH fusion, wherein the fused domains are linked together by a flexible peptide linker. The linker may have a length of about 1 to 20 amino acids, preferably of between about 2 and 7 amino acids. Typically, small amino acids like glycine may be used for the linker. They may also be combined with other amino acids. The term "minibody" as used herein refers to a size reduced antibody, e.g. an antibody comprising solely variable domains or lacking constant domains or comprising the variable heavy domain.

Preferred antibodies are typically monospecific. In certain specific embodiments, the antibody may also be bi-specific or of a greater multi-specificity. Multi-specific antibodies may be specific for different epitopes.

The term "modified antibody" as used herein refers to derivatives which are modified, for instance by the covalent attachment of any type of molecule to the antibody such that said covalent attachment does not prevent the antibody from specifically binding to its target or from generating an anti-idiotypic response. Typical examples of such modifications are glycosylation, acetylation, biotinylation, PEG-ylation, phosphorylation, amidation, derivatization by known protecting/blocking groups, proteolytic cleavage, linkage to a cellular ligand or other protein, etc. Chemical modifications may be carried out by known techniques, including specific chemical cleavage, acetylation, formylation etc. Additionally, the derivative may contain one or more non-classical amino acids.

Antibody "fragments" as used herein are antibody sub-structures which recognize specific epitopes and which may be generated by known techniques. For example, Fab and F (ab')2 fragments may be produced by proteolytic cleavage of immunoglobulin molecules, using enzymes such as papain (to produce Fab fragments) or pepsin (to produce F (ab') 2 fragments).

The term "non-immunoglobulin protein" refers to a group of highly affine proteins which are capable of specifically binding to a target, but do not comprise immunoglobulin domains or elements.

The non-immunoglobulin proteins may offer several distinct mechanisms of binding and preferably have a similar affinity for target structures as antibodies. Examples of non-immunoglobulin proteins which are preferably used in the context of the present invention include protein structures comprising ankyrin-repeats. Typically, in designed "ankyrin-repeat proteins" (DARPins) three, four or preferably five repeat ankyrin motifs are present. These may form a stable protein domain with a large potential target interaction surface. Further envisaged are affimer molecules. "Affimers" are small, engineered proteins that bind to target proteins with affinity in the nanomolar range. They are derived from the cysteine protease inhibitor family of cystatins. Affimer proteins display two peptide loops that can be randomized to bind to desired target proteins. Stabilization of the two peptides by the protein scaffold constrains possible conformations that the peptides can take, thereby increasing the binding affinity and specificity compared free peptides. Another example of a highly affine non-immunoglobulin is an aptamer. The term "aptamer" as used herein refers to a nucleic acid and/or peptide that specifically binds to a target. Aptamers are typically selected from pools of nucleic acids and/or peptides for their selective binding properties. A further preferred example of a highly affine non-immunoglobulin protein is an "affibody" molecule, i.e. a protein based on the Z domain (the immunoglobulin G binding domain) of protein A. In contrast to antibodies, affibody molecules are typically composed of alpha helices and lack disulfide bridges. They may be expressed in soluble and proteolytically stable forms in various host cells. Affibody molecules may further be fused with other proteins. The group of highly affine non-immunoglobulin proteins according to the present invention also comprises adnectins. "Adnectins" are based on the structure of human fibronectin, in particular its extracellular type III domain, which has a structure similar to antibody variable domains, comprising seven beta sheets forming a barrel and three exposed loops on each side corresponding to the three complementarity determining regions. Adnectins typically lack binding sites for metal ions and central disulfide bonds. They are approximately 15 times smaller than an IgG type antibody and comparable to the size of a single variable domain of an antibody. Adnectins may be customized in order to generate and/or increase specificity for targets by modifying the loops between the second and third beta sheets and between the sixth and seventh sheets. A further preferred example is the antibody mimetic anticalin, which is derived from human lipocalin.

"Anticalins" typically have the property of binding protein antigens, as well as small molecule antigens. They are composed of a barrel structure formed by 8 antiparallel beta sheets, connected by loops and an attached alpha helix. Mutagenesis of amino acids at the binding site may allow for changing of affinity and selectivity of the molecule. Another preferred example is "affilin", i.e. a genetically engineered protein with the ability to selectively bind antigens, which is structurally derived from gamma-B crystallin or from ubiquitin. Affilins are typically constructed by modification of near-surface amino acids of gamma-B crystallin or ubiquitin and isolated by display techniques such as phage display. The molecular mass of crystallin and ubiquitin based affilins is typically about one eighth or one sixteenth of an IgG antibody, respectively. This may lead to heat stability up to 90°C and an improved stability towards acids and bases. The group of highly affine non-immunoglobulin proteins also comprises "avimers", i.e. artificial proteins that are able to specifically bind to certain antigens via multiple binding sites. Typically, the individual avimer sequences are derived from A domains of various membrane receptors and have a rigid structure, stabilized by disulfide bonds and calcium. Each A domain can bind to a certain epitope of the target molecule. The combination of domains binding to different epitopes of the same target molecule may increase affinity to the target. Further preferred examples include "knottins", i.e. small disulfide-rich proteins characterized by a special disulfide through disulfide knot. This knot is typically obtained when one disulfide bridge crosses the macrocycle formed by two other disulfides and the interconnecting backbone (disulfide III-VI goes through disulfides I-IV and II-V) . Knottin peptides could be shown to bind with high affinity (about 10 to 30 nmol/L) to integrin receptors. Also envisaged are "fynomers", i.e. Fyn SH3-derived proteins. Fyn is a 59-kDa member of the Src family of tyrosine kinases. The Fyn SH3 domain comprises 63 residues, and its amino acid sequence is fully conserved among man, mouse, rat, and monkey. Fynomers are typically composed of two antiparallel beta sheets and contain two flexible loops (RT and n-Src loops) to interact with other proteins or targets. Another preferred example of a highly affine non-immunoglobulin molecule is a phylomer peptide. "Phylomer" peptides are bioactive fragments of naturally occurring proteins that are encoded in the genomes of evolutionary diverse microbes, which are partially sourced from extreme environments and may have evolved over billions of years, providing a multitude of distinct and stable structures capable of binding to biological molecules. A further, preferred example of a highly affine non-immunoglobulin molecule is an artificial antibody mimic. The term "artificial antibody mimic" as used herein refers to a polymeric organic or inorganic structure which specifically binds a target molecule, typically with a binding affinity in the range of an antibody. Examples of such artificial antibody mimics are molecularly imprinted polymers (MIPs). The principle of molecular imprinting relies on the formation of a host-guest complex between a template molecule of interest and one or more functional monomers. The templates allowed to associate with the functional monomers in an appropriate solvent, and a plethora of noncovalent interactions may be formed. In addition, a cross-linking agent may be added, which may provoke subsequent polymerization. Thereby typically a rigid and highly cross-linked polymer matrix is formed which encapsulates the template molecule within. Subsequently, the template molecule may be removed so that an imprint remains, which is complimentary to the original molecule. Additionally envisaged is the employment of kunitz domain peptides. "Kunitz domains" are the active domains of Kunitz-type protease inhibitors. They typically have a length of about 50 to 60 amino acids and a molecular weight of 6 kDa. Examples of Kunitz-type protease inhibitors are aprotinin, Alzheimer's amyloid precursor protein (APP) , and tissue factor pathway inhibitor (TFPI) . Kunitz domains are stable as standalone peptides and are able to recognize specific targets such as protein structure and may accordingly be used for the design of highly affine molecules which are able to bind detection moieties according to the invention.

According to a specific embodiment of the invention the free first interactor is used to extend the dynamic range of an assay.

It is preferred that the free first interactor, e.g. a specifically binding molecule as defined herein above, is provided as dried reagent in said one or more transport or reaction zones. The free first interactor may, for example, be prepared as lyophilized antibody, e.g. from a buffer such as PBS or another suitable liquid or it can, in alternative embodiments, be prepared by drying of a dosing solution or within a dosing buffer. The interactor may, for usage, be reconstituted by the addition of a suitable liquid, e.g. a liquid as present in a fluid sample. Further, pH and ionic strength in the liquid may be adjusted according to the skilled person's knowledge. The dried interactor may, in certain embodiments, further be mixed with stabilizing sugars or proteins such as BSA. It is preferred that the stabilization in dried form is implemented by using sugar molecules.

In further embodiments, the free first interactor, e.g. a specifically binding molecule as defined herein above, is provided as liquid reagent in said one or more transport or reaction zones. The free first interactor may, for example, be provided in a suitable solution such as a buffer solution or an aqueous solution. Said solution may additionally comprise stabilizing factors such as proteins or sugars or be adjusted with respect to its pH or ionic strength.

It is particularly preferred that said free first interactor is not immobilized on a bead or on a surface of the cartridge. Thereby, an independent and rapid interaction of the free first interactor with a target can be achieved which reduces the amount or concentration of approachable target for detecting interactions, e.g. by the second interactor, preferably at specific sites of the cartridge such as a reaction zone.

In particularly preferred embodiments, said free first interactor is provided, e.g. as liquid or dried free interactor, at the place of first contact of the fluid sample with reagents in the cartridge. Such a place of first contact may, for example, be a landing zone as defined herein, or a transport zone or a reaction zone. The interactor may further be provided in more than one of such zones. It is particularly preferred that the target, when entering the cartridge within the fluid sample, is first interacted with the free first interactor before an interaction with a second interactor can take place. In certain embodiments, the present invention further envisages that the target is exposed to the first and second interactors at the same time such that an equilibrium state of binding can be achieved, e.g. resulting in a situation in which a certain fraction of the target may bind to the first and another fraction of the target may bind to the second interactors. This may, for example, be performed when target concentration is low in order to prevent saturation of the target with first interactors.

In a further aspect, the present invention relates to a system comprising a handheld device and the cartridge for the uptake and analysis of a fluid sample as described herein. The handheld device is capable of operating the cartridge. The term "device capable of operating the cartridge" as used herein refers to a device which is designed as reader or control unit. It may, accordingly, for example, provide pneumatic and/or electrical connections to the cartridge allowing to induce in the cartridge sample movements, reactions with the sample, measurement of parameters etc. Furthermore, the reader or control functionality of the device may additionally comprise sensors or detecting elements allowing to accumulate measured values in the cartridge. In further embodiments, the device may control environmental conditions of the cartridge, e.g. temperature, humidity etc. It may also control reactions by applying magnetic fields or focused irradiation. Also, a digital interface, e.g. comprising WiFi and/or Bluetooth and/or WLAN connectivity, may be provided within the handheld device, i.e. the system. Obtained measurement results may accordingly be transmitted to remote server structures or cloud servers or be transmitted to further local devices such as desktop computer, tablets or the like. In further embodiments, detector elements and assessment units necessary for the detection of the processing state or functioning of the cartridge may be provided by the device.

Both elements of the system, i.e. the handheld device and the cartridge, may be connected in a push fit fashion, e.g. as cradle and plug-in module. The handheld device may accordingly be provided with opening or receptacle structures, allowing the connection or introduction of a cartridge. The physical separation of both elements of the system according to the present invention provides the advantage that the same device may be used for multiple analyses, while the cartridge may comprise disposable, non-reusable or non-expensive elements such as chemical reactants or assay components etc. A cartridge is in a preferred embodiment thus envisaged as a single use or disposable product. In a particularly preferred embodiment, the device is an Atellica^{®} Point-of-Care device, e.g. an Atellica^{®} VTLi Patient-side Immunoassay Analyzer.

In a further aspect, the present invention relates to a method of reducing the active concentration of a target in a fluid sample, which method comprises the step of contacting at least one free first interactor of said target with the fluid sample to allow the binding of said at least one free first interactor to said target, thereby reducing the active concentration of the target in the sample. The terms "target" and "free first interactor" have been defined herein above. The term "active concentration of the target" as used herein relates to the amount of target molecules which can be detected or bound by a suitable interactor, preferably a second or further interactor as defined herein, which is designed for detecting and/or capturing the target. Such a second or further interactor may, for example, be immobilized at a certain site or in a certain zone of the cartridge or be labeled as described herein, thus allowing for a qualitative or quantitative analysis of the target as described herein. The active concentration may accordingly be understood as the total amount of target in the fluid sample which was entered into the cartridge minus the amount of target which is bound by the free first interactor as defined herein. The active concentration may, in certain embodiments, be adjusted or set by the amount or concentration of free first interactor in the cartridge. This may be adapted to the typical concentration of target in a sample or other factors having an influence on said concentration. In certain embodiments of the present invention the invention the active concentration of the target in the sample may be reduced by 5 to 95%, preferably by 10 to 90%, more preferably by 25 to 75%.

In a further aspect, the invention relates to a method of analyzing a fluid sample with respect to the presence and/or quantity of a target, the method comprising a step of reducing the active concentration of the target in the fluid sample according to the method of reducing the active concentration of a target in a fluid sample as described herein and wherein the method comprises the additional step of analyzing said fluid sample with respect to the presence and/or quantity of the target. The analysis may, for example, be performed as qualitative, semi-qualitative or quantitative analysis as described herein above. The analysis may, in further embodiments, be an or be based on an assay as described above, e.g. a biological or biochemical assay, a kinetic assay or an immunoassay. It is preferred that the analysis is performed by using a second, and, optionally, a further interactor which second and/or further interactors are designed for detecting and/or capturing the target as defined herein. In certain embodiments, said analysis may be performed simultaneously with the binding of the free first interactor to the target as defined herein above.

In an embodiment of the method the free first, the second and/or any further interactor of said target is a molecule specifically binding to said target, preferably an antibody, an intrabody, an antibody substructure, a modified antibody, or a fragment thereof; or a non-immunoglobulin protein selected from the group of: designed ankyrin-repeat protein (DARPin), affimer, aptamer, affibody molecule, adnectin, anticalin, affilin, avimer, knottin, fynomer, phylomer, artificial antibody mimic and kunitz domain peptide as defined herein above.

In a further preferred embodiment, the method comprises a step of calibrating one or more reagents. The calibration may, for example, be based on the usage of a sample containing a known concentration of the target to be detected in the assay. It may correspondingly be used to create a calibration curve to calculate the concentration of the target in unknown specimens by linking the signal to a specific value on the curve. In a specific embodiment, the calibration may be performed by measuring samples with known concentration and recording the output signal for each sample. Thereby a calibration curve can be obtained which can be used to link the measurement result of an unknown sample to a dose or specific value on this curve. Further steps may involve the comparison of obtained values with previously obtained or reference values, e.g. derivable from a suitable database.

In further preferred embodiments of the methods according to the invention, the step of binding said free first interactor to said target is performed for about 1 to 15 min as of the first contact of the fluid sample with the at least one free first interactor, e.g. for a period of time of 1 min, 2 min, 3 min, 4 min , 5 min, 6 min, 7 min, 8 min, 9 min, 10 min, 11 min, 12 min, 13 min, 14 min, 15 min or any value in between the mentioned values.

It is preferred that the incubation step including the interaction of the target with a first, second and, optionally, further interactor, takes place simultaneously such that an equilibrium can be reached between the target molecule and the interactor(s).

In very specific embodiments, the incubation step may alternatively exclude an interaction with a second or further interactor, i.e. the detection of the target. The exclusion may, for example, be implemented by a transient closure of certain zones or chambers of the cartridge, thus preventing the fluid sample from leaving the zone of contact with the free first interactor.

In further embodiments, the contacting/binding is performed continuously upon first contact of the fluid sample with reagents. The term "continuously" as used herein refers to the provision of one or more free first interactors for a contacting with targets present in the fluid sample during all or essentially all steps of transport and analysis in the cartridge. For example, by providing said free first interactors in all or essentially all transport zones and in all or essentially all reaction zones a continuous exposure of the target to specifically binding free first interactors can be achieved.

In preferred embodiments, the method is performed with the device as described herein, and/or the cartridge as described herein and/or the system as described herein.

In yet another aspect, the present invention relates to the use of the cartridge as defined herein or the system as defined herein for molecular diagnosis, immunological assays, biological or chemical sample analysis, body fluid sample analysis, preferably blood sample analysis, saliva sample analysis, intestinal fluid sample analysis, eye fluid sample analysis, urine sample analysis, pharmacology, food analysis, environmental, veterinary or forensic analysis. The diagnostic or analytical procedures may comprise, in certain embodiments, the provision of the analytical conclusion or results in a corresponding report. Such a report may be provided in any suitable manner or form, e.g. as electronic file, as electronic file distributed or accessible over the internet, e.g. provided in a cloud or deposited on a server, or web-based, e.g. provided on a suitable website. Alternatively, the report may be provided in paper form.

The report may be provided and thus drafted in a corresponding form, to a subject or another person associated with the subject, a scientist or group of scientists, a judicial authority, a hospital or clinic, a medical practitioner, or a government office (including information relevant for these entities) . The report may accordingly be redacted, modified, extended or adjusted to the above specified recipient. The report may further be provided as reading on a display.

The figure is provided for illustrative purposes. It is thus understood that the figure is not to be construed as limiting. The skilled person in the art will clearly be able to envisage further modifications of the principles laid out herein.

### EXAMPLE 1: Quantitative measurement of C-reactive protein (CRP)

A homogeneous immunoassay is conducted in a self-contained, single-use cartridge. Fluid samples containing CRP target are mixed and incubated with different amounts of free C6cc antibody with specificity for CRP protein in a concentration range of 0.25-0.5 mg/mL and finally applied to the cartridge. In the reaction chamber of the cartridge, the mixed sample is contacted with superparamagnetic nanoparticles to which C6cc antibodies are covalently bound. The nanoparticles and the mixed sample containing free C6cc antibodies are incubated in the reaction chamber for 7.5 min to allow capture of CRP present in the sample. CRP-protein present in the sample will bind to both interactors, the C6cc antibody on the nanoparticles as well as the free C6cc antibody in solution, thereby effectively diluting the available CRP-protein concentration. Following incubation, magnetic force attracts the metal nanoparticles to the sensor surface of the reaction chamber where captured CRP species affix to detection antibodies.

After the nanoparticle-CRP-antibody complex is immune-affixed to the sensor surface, application of magnetic force at the opposite surface removes free nanoparticles. To detect immune-affixed complexes, a light beam focuses at an incidence angle below the critical angle at the sensor surface so the light is totally reflected. When no complexed nanoparticles are affixed on the sensor surface, all the light is reflected and read by the image detector. When complexed nanoparticles are bound to the surface, light is scattered and/or absorbed, which reduces the intensity of the reflected beam reaching the optical image detector in proportion to the sample's CRP concentration. This signal is converted to a CRP result. Cartridges were measured over a concentration range of 0-1000 mg/L CRP-protein. The results are shown in FIG. 1.

## Claims

1. A cartridge for the uptake and analysis of a fluid sample with respect to the presence and/or quantity of a target, the cartridge comprising one or more reaction zones and one or more transport zones, which zones are designed for contacting reagents with a sample, wherein said zones comprise as reagent at least one free first interactor of said target.

2. The cartridge of claim 1, wherein the free first interactor of said target is a molecule specifically binding to said target.

3. The cartridge of any one of claims 1 and 2, comprising as reagent a second and, optionally, a further interactor of said target which second and further interactors are designed for detecting and/or capturing the target and wherein said free first interactor (i) binds in the same binding region of the target as the second and/or the further interactors ; or (ii) binds at any region of the target, and thereby prevents the binding of said second and/or further interactors.

4. The cartridge of any one of claims 2 and 3, wherein said specifically binding molecule is an antibody, an antibody substructure, a modified antibody, or a fragment thereof; or a non-immunoglobulin protein selected from the group of: designed ankyrin-repeat protein (DARPin), affimer, aptamer, affibody molecule, adnectin, anticalin, affilin, avimer, knottin, fynomer, phylomer, artificial antibody mimic and kunitz domain peptide.

5. The cartridge of any one of claims 1 to 4, wherein the free first interactor is provided as dried reagent in said zones, wherein the free first interactor is not immobilized on a bead or a surface of the cartridge.

6. The cartridge of any one of claims 1 to 4, wherein the free first interactor is provided as liquid reagent in said zones.

7. The cartridge of any one of claims 1 to 6, wherein said free first interactor is provided at the place of first contact of the fluid sample with reagents in the cartridge, preferably provided in a mixture with a buffer or buffer system.

8. A system comprising a handheld device capable of operating the cartridge of any one of claims 1 to 7 and the cartridge as defined in any one of claims 1 to 7.

9. A method of reducing the active concentration of a target in a fluid sample, comprising the step of contacting at least one free first interactor of said target with the fluid sample to allow the binding of said at least one free first interactor to said target, thereby reducing the active concentration of the target in the sample.

10. A method of analyzing a fluid sample with respect to the presence and/or quantity of a target, the method comprising a step of reducing the active concentration of a target in a fluid sample according to the method as defined in claim 9, wherein the method comprises the additional step of analyzing said fluid sample with respect to the presence and/or quantity of the target, preferably by using a second and, optionally, a further interactor which second and further interactors are designed for detecting and/or capturing the target.

11. The method of any one of claims 9 and 10, wherein said free first interactor, the second and/or any further interactors of said target is a molecule specifically binding to said target, preferably an antibody, an antibody substructure, or a modified antibody, or a fragment thereof; or a non-immunoglobulin protein selected from the group of: designed ankyrin-repeat protein (DARPin), affimer, aptamer, affibody molecule, adnectin, anticalin, affilin, avimer, knottin, fynomer, phylomer, artificial antibody mimic and kunitz domain peptide.

12. The method of any one of claims 9 to 11, additionally comprising a step of calibrating one or more reagents, preferably in a cartridge as defined in any one of claims 1 to 7.

13. The method of any one of claims 9 to 12, wherein the binding of said at least one free first interactor to said target is performed for about 1 to 15 min as of the first contact of the fluid sample with the at least one free first interactor.

14. Use of the cartridge of any one of claims 1 to 7 or of the system of claim 8 for molecular diagnosis, immunological assays, biological or chemical sample analysis, body fluid sample analysis, preferably blood sample analysis, saliva sample analysis, intestinal fluid sample analysis, eye fluid sample analysis, urine sample analysis, pharmacology, food analysis, environmental, veterinary or forensic analysis.

15. The cartridge of any one of claims 1 to 7, the system of claim 8, or the method of any one of claims 9 to 13, wherein the fluid sample is a blood or blood plasma sample, a saliva sample, an intestinal fluid sample, a urine sample or an eye fluid sample.
